# EUROPEAN PATENT SPECIFICATION

(11) **EP 3 666 276 B1**
(45) Date of publication and mention of the grant of the patent: **16.09.2026**
(21) Application number: 18212759.7
(22) Date of filing: 14.12.2018
(51) Int. Cl.: A61K 31/5685, A61P 35/00

(54) **MEDICATION AGAINST ESTROGEN-RECEPTOR BETA (ERBETA) POSITIVE BREAST TUMOR**
MEDIKAMENT GEGEN ÖSTROGEN-REZEPTOR-BETA (ERBETA)-POSITIVEN BRUSTTUMOR
MÉDICAMENT CONTRE LA TUMEUR POSITIVE DU RÉCEPTEUR D'OESTROGENE BETA (ERBETA)

(43) Date of publication of application: 17.06.2020
(73) Proprietor: dcic Biopharmaceutical Limited, Hong Kong (HK)
(72) Inventor: WIELAND, Heinrich, 79271 St. Peter (DE)
(74) Representative: Prüfer & Partner mbB Patentanwälte · Rechtsanwälte

(56) References cited:
- EP-B1- 2 018 170
- CA-A1- 2 324 077
- US-A1- 2018 169 112
- US-B2- 8 343 948
- MARCELO MADEIRA ET AL: "Estrogen receptor alpha/beta ratio and estrogen receptor beta as predictors of endocrine therapy responsiveness-a randomized neoadjuvant trial comparison between anastrozole and tamoxifen for the treatment of postmenopausal breast ca", BMC CANCER, BIOMED CENTRAL, LONDON, GB, vol. 13, no. 1, 18 September 2013 (2013-09-18), pages 425, XP021163528, ISSN: 1471-2407, DOI: 10.1186/1471-2407-13-425

## Description

### Field of the invention

The present invention relates to the field of medicine, and refers to the steroid compound 4-hydroxyandrost-4-ene-3,17-dione (4-OHA) as a prodrug for use in the treatment of a patient suffering from estrogen-receptor β (ERβ) positive breast tumour. The present invention further relates to a pharmaceutical composition comprising said steroid compound for topical application, and the use of said steroid compound in a pre-surgical topical treatment of ERβ-positive breast cancer tissue.

### Description of the background art

After colorectal cancer breast cancer is the most frequent cancer disease in the western world despite the fact that it affects almost exclusively women. In Germany, breast cancer amounts to about 20% of all cancer types diagnosed in women.

Up to now, the therapy in most of the cases consists of surgical removal of the tumor (lumpectomy) or complete removal of the affected breast if the size of the tumor prevents breast conserving therapy. In both cases the operation (surgical removal) is followed by drug therapy. A particular complication of breast carcinomas is the high potential of metastatic spread into other organs particularly liver, brain, bones and the skin. Therefore, the operation is followed by drug therapy (adjuvant therapy). This therapy is also aimed at preventing local recurrence of the disease. If the removed tumors were exhibiting estrogen receptors, antiestrogens are used (Tamoxifen or aromatase-inhibitors. The lack of estrogen receptors leads to a postoperative drug therapy using cytostatic drugs.

Tamoxifen binds to the estrogen receptor (ER) and blocks the growth promoting effect of estrogens. Aromatase-inhibitors block the final step in the production of estrogens from androgenic precursors (c-19-steroids). They either bind irreversibly to the active site of the enzyme (aromatase-inactivators, i.e. steroidal aromatase-inhibitors: 4-hydroxyandrostenedione (Formestane = Lentaron^{®}), or Exemestane (Aromasin^{®})) or inhibit competitively the cytochrome p 450 part of the enzyme (nonsteroidal aromatase-inhibitors: Anastrozol (Arimidex^{®}) and Letrozol (Femara^{®}).

If the breast cancer exhibited an overexpression of the Her2neu gene, antibodies against this cell-surface protein are given intravenously such as e.g. Trastuzumab (Herceptin^{®}).

Basic and clinical data indicate that aromatized metabolites of androgens, i.e. the estrogens, are the hormones involved in the pathogenic cellular changes associated with the growth of some hormone-dependent cancers, such as breast, endometrial and ovarian carcinomas. Aromatase has recently been recognized as an enzyme that is capable to synthesize estradiol directly in tumours.

Endogenous estrogens are ultimately formed from either androstenedione or testosterone as immediate precursors. The local production of estrogens in the breast tissue, especially in the tumour cells itself, had been regarded to be of great importance for the therapy of the disease. In this context, aromatization of the steroidic ring A performed by the enzyme aromatase, and its inhibition had been considered to play a major role in anti-breast cancer approaches.

Therefore, conventional therapeutic approaches to address breast cancer in women - besides other concepts based on estrogen receptor antagonists or therapeutic antibodies -focused on an aromatase-inhibiting action itself. Accordingly, steroidal substances which had been reported to be endowed with an aromatase-inhibiting action have been described, for example Δ¹-testololactone [U.S. Pat. 2,744,120], 4-hydroxy-androst-4-ene-3,17-dione and esters thereof [see, for example, U.S. Pat. 4,235,893], 10-(1,2-propadienyl)-estr-4-ene-3,17-dione (U.S. Pat. 4,289,762], 10-(2-propynyl)-estr-4-ene-3,17-dione [J. Amer. Chem. Soc., 103, 3221 (1981) and U.S. Pat 4,322,416], 19-thioandrostene derivatives (Europ. Pat. Appl. 100566), androsta-4,6-diene-3,17-dione, androsta-1,4,6-triene-3,17-dione [G.B. Pat Appl. 2,100,601A] and androsta-1,4-diene-3,17-dione [Cancer Res. (Suppl.) 42,3327 (1982)].

Clinical studies conducted with estrogen-receptor (ER) antagonists and aromatase inhibitors however showed that these compounds were not able to exert a significant and sufficient inhibitory effect on tumour proliferation and/or growth. For example, Tamoxifen^{®} based therapy suffers from drawbacks such as tachyphylaxy (therapy failure) and the fact that its effects in some cells are estrogen-like (e.g. risk for thrombosis and endometrial cancer) being the reason that Tamoxifen^{®} cannot be administered for a period longer than 3 to 5 years. Newer drugs such as aromatase inhibitors can in principle only be used in post-menopausal women and, furthermore, are only effective on ER-positive tumours.

It could be shown (J. Szelei et al., "Androgen-Induced Inhibition of Proliferation in Human Breast Cancer MCF7 Cells Transfected with Androgen Receptor", Endocrinol., 138, no. 4, 1406-1412, 1997) that stable transfected cells expressing androgen receptor (AR) acquire the ability to respond to androgen by evoking an end of cellular proliferation. A more recent report (J. Ortmann et al., "Testosterone and 5α-dihydrotestosterone inhibit in vitro growth of human breast cancer cell lines", Gynecol. Endocrinol. 2002; 16:113-120) shows that testosterone and 5α-dihydrotestosterone (DHT) can inhibit cell proliferation in certain cell lines. Testosterone itself has a similar effect. Its clinical usefulness however is hampered by the fact that it undergoes rapid metabolization to either estradiol or dihydrotestosterone (DHT). The local formation of estradiol from testosterone within the breast cancer by local aromatase activity promotes cancer-growth.

On the other hand, AR itself had been regarded as a modulator of processes involved in differentiation, homeostasis, and development of male secondary characters. Thus, AR-directed treatment schemes had been focused on androgen-related diseases in men, including hypogonadism, male infertility, prostate cancer, delay of puberty, hirsutism, androgen-deficient diseases, androgen replacement in aging men, and male pattern baldness. In this context, a disclosure of US 2,762,818A does not go beyond using 4-hydroxytestosterone and its esters, based on their androgenic and anabolic properties, to treat an androgen deficiency status itself. There was however neither a purpose nor a finding which would suggest an activity on breast cancer cells or on effects from which an efficacy against the disease conditions could have been deduced. Further, US2003/0229063A addresses low androgen to estrogen ratios in men (leading to endocrine disorders) and only for this purpose attempts to make use of 4-hydroxytestosterone based on an asserted aromatase-inhibiting effect alone.

WO2007/131737 focusses on the treatment of *inter alia* breast cancer, and in this context refers to the targeting of the androgen receptor (AR) with a steroid compound.

As already discussed above, some breast cancer tumors are sensitive towards estrogens. Estrogens exert their actions via specific nuclear protein receptors, namely estrogen receptor alpha (ERα) and estrogen receptor beta (ERβ).

Hence, a lot of studies have been carried out in order to elucidate the role and nature of ERα and ERβ. In this regard, it could be shown that in most cases of breast cancer, ERα expression dominates ERβ expression. During progression of the disease, the expression of ERβ is reduced, in parallel to the gain of malignancy.

Based on cell culture system studies, it could be shown that these two subtypes of estrogen receptors mediate the actions of estrogens, and that the relative levels of ERβ and ERα in breast cancers are likely to impact cell proliferation and the activities of diverse signalling pathways and their responses to ER ligands (Endocrinology, Volume 147, Issue 10, 1 October 2006, Pages 4831-4842). Further, it could be shown that ERβ acts as tissue-specific tumor-suppressor with anti-proliferative properties (Clin. Pharmacol. Ther. 2011 Jan; 89(1):44-55). Moreover, activation of ERβ can further result in a disruption of the signal pathway mediated by ERα by the generation of heterodimers comprising ERα and ERβ (ERα:ERβ heterodimer). In normal, that is, non-diseased breast tissue, the expression of ERα predominates, whereas in diseased tissue, that is, breast cancer tissue, the expression of ERβ dominates. It is currently believed that ERα drives the estrogen-mediated proliferation, whereas ERβ is a counterpart that limits or even completely prevents this estrogen-mediated proliferation of ERα (Mol. Endocrinol. 2003, Feb; 17(2): Pages 203-8; Lindberg et al.).

US 2018/0169112 refers to the use of a pharmaceutical medication comprising an aromatase inhibitor, preferably a steroidal aromatase inactivator and an antioxidant as effective ingredients for the treatment of sex-hormone-dependent diseases such as breast cancer.

CA 2 324 077 refers to the use of a steroidal aromatase inhibitor for topical use for preventing and/or treating a mammary carcinoma.

US 8,343,948 B2 refers to a method of prophylaxis and/or treatment of mammary carcinoma which involves the topical application of a therapeutically or prophylactically effective amount of a steroidal aromatase inhibitor locally, to an area of a patient in need of treatment.

EP 2 018 170 B1 refers to the use of 4-hydroxytestosterone for the prophylaxis and treatment of e.g. cancers.

Further disclosed is the use of anastrozole in the treatment of ERβ positive breast cancer (BMC Cancer, Volume 13, No. 18 September 2013, Page 425).

As can be seen from the above, in the field of breast cancer, the therapeutic concepts known so far either focus on the inhibition of aromatase alone, or on the binding on the androgen receptor (AR) alone. Moreover, in addition to the treatment of breast cancer by means of inhibiting aromatase, or binding the AR, the therapy additionally comprises, in most of the cases, a surgical removal of the tumor, or a complete removal of the affected breast, if the size of the tumor prevents a breast conserving therapy.

Such therapies, and in particular the partial or even complete removal of the affected breast, are a great physical and mental burden for the patients.

Thus, there is a need, and thus it is an object of the present invention, to provide an effective therapeutic approach for treating patients suffering from a certain type of breast cancer, in particular from ERβ positive breast cancer. In particular, it is the object of the present invention to provide an effective therapeutic approach that allows for the reduction of the tumor size to a size that, in turn, allows for a breast conserving therapy, that is, a therapy that prevents the complete surgical removal of the affected breast.

### Summary of the invention

Various aspects, advantageous features and preferred embodiments of the present invention as summarized in the following items, respectively alone or in combination, contribute to solving the object of the invention.
1. The present invention refers to steroid compound 4-hydroxyandrost-4-ene-3,17-dione (4-OHA) of formula I 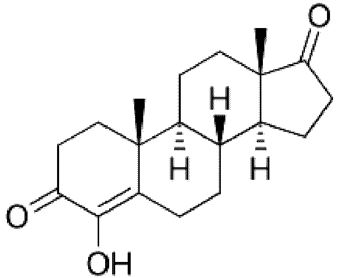
   Formula I, or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group,
   for use in a method of treating a patient suffering from estrogen-receptor β (ERβ) positive and optionally androgen receptor (AR) positive breast tumor, wherein said steroid compound is applied topically to the patient.

In a preferred embodiment, the patient suffers from an ERβ positive and AR positive breast tumor.

Within the meaning of the present invention, the term "prodrug" defines a medication or compound, that, after administration, is metabolized (that is, converted within the body) into a pharmacologically active substance.

Within the meaning of the present invention, the term "ester" denotes suitable ester groups, such as substituted or unsubstituted hydrocarbon groups (optionally the carbon atoms of the hydrocarbon groups may also be substituted by 1-3 heteroatoms (O, S, N)). In particular, the ester groups can be straight chain, branched chain, cyclic and/or aromatic hydrocarbon groups or acyl groups like aroyl groups, such as formyl, acetyl, propionyl, butyryl, pentanoyl, hexanoyl, heptanoyl, and benzoyl, without being limited thereto. The hydrocarbon groups may have 1-12 carbon atoms and can be substituted or unsubstituted with substituents such as halogens. The carbon atoms of the hydrocarbon group may also be substituted by 1-3 heteroatoms (O, S, N), e.g., to form heteroaromatic groups.

In the present invention, the ester is formed with the 4-OH (hydroxyl) group.

Whenever reference is made to 4-OHA in the present invention, an ester thereof is also comprised by this term.

The esters according to the above definition are used as prodrugs; upon administration the ester group is metabolized, leaving the uncoupled 4-OH group.

2. In a preferred embodiment, the present invention refers to the steroid compound 4-OHA or esters thereof for use according to item 1, wherein the treatment takes place prior to a planned surgical removal of breast tumor tissue, and/or wherein the treatment is for reducing the breast tumor tissue.

3. In a further preferred embodiment, the steroid compound 4-OHA or esters thereof for use according to item 1 or 2, is metabolized to a pharmacologically active substance upon topical application which has a hydroxyl-group in at least C4-position and C17-position.

4. In a further preferred embodiment, the steroid compound 4-OHA or ester thereof for use according to any of items 1 to 3, is metabolized to a pharmacologically active substance which has a higher binding affinity towards estrogen-receptor beta (ERβ) as compared with its binding affinity towards estrogen-receptor alpha (ERα), which are both expressed by the breast tumor tissue, wherein said treatment results in agonistic binding of ERβ, preferably wherein said pharmacologically active substance exhibits a binding affinity towards ERβ that is at least 3 times, preferably at least 4, 5, or 6 times, more preferably at least 9 times and most preferably at least 10 times higher compared to the binding affinity towards ERα.

It is additionally preferred that the pharmacologically active substance has binding affinity towards the androgen receptor that is expressed by the breast tumor tissue (in addition to the ERβ).

5. Further disclosed herein is the steroid compound 4-OHA or ester thereof for use according to any one of items 1 to 4, which is a pharmacologically active substance which comprises, preferably is, 4-hydroxy-5-alpha-androstane-3-beta,17beta-diol (4-OHBA) or 4-hydroxytestosterone (4-OHT), preferably the pharmacologically active substance is 4-OHBA.

6. The present invention further refers to a pharmaceutical composition suitable for topical application comprising the steroid compound 4-hydroxyandrost-4-ene-3,17-dione (4-OHA) of formula I 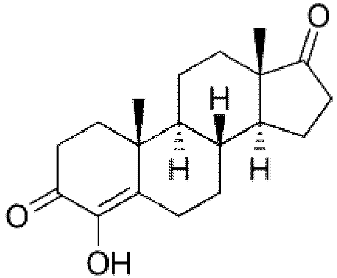 Formula I or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group,
and pharmaceutically acceptable carrier(s) and/or excipient(s), for use in the treatment of a patient suffering from estrogen-receptor β (ERβ) positive breast tumor and optionally androgen receptor (AR) positive breast tumor, wherein the treatment takes place prior to a planned surgical removal of the breast tumor tissue.

In a preferred embodiment, the patient is suffering from ERβ positive and AR positive breast tumor.

7. In a preferred embodiment, the pre-surgical treatment referred to in item 6 comprises the topical application of the pharmaceutical composition in an amount effective for reducing the breast tumor tissue.

8. In a further preferred embodiment, the pre-surgical treatment referred to in item 6 or 7 is carried out over a period of time that is sufficient for effecting the reduction of the breast tumor tissue to a size that allows for the breast-preserving surgical removal of the breast tumor tissue.

In a preferred embodiment, the pre-surgical treatment is carried out for a period of time of up to 6 months, or up to 5, 4, 3, or 2 months.

It is also possible that a reduction of the breast tumor tissue to a size that allows for the breast-preserving surgical removal can be effected after a pre-surgical treatment of 11 weeks, or 10, 9, 8, 7, 6, 5, 4, 3, or 2 weeks.

9. In a further preferred embodiment, the tumor tissue referred to in any one of items 6 to 8 is significantly reduced upon completion of the treatment, preferably the tumor tissue is reduced by at least 20 vol.-%, preferably by at least 30 vol.-%, more preferably by at least 40 vol.-%, even more preferably by at least 50 vol.-%, compared to the size of the breast tumor tissue at the beginning of the treatment, as determined by ultrasound, X-rays, or mammography. In a preferred embodiment, the size of the breast tumor tissue is determined by mammography.

10. In a further preferred embodiment, the pre-surgical treatment referred to in any one of items 6 to 9 starts no later than 6 months, or no later than 5, 4, 3, or 2 months, prior to the planned surgery, that is, the breast-preserving surgical removal of the breast tumor tissue.

It is also possible that the pre-surgical treatment starts no later than 11 weeks, or 10, 9, 8, 7, 6, 5, 4, 3, or 2 weeks, prior to the planned surgery.

It is also possible that the pre-surgical treatment starts no later than 8 weeks prior to a planned surgery, preferably no later than 6 weeks or 5 weeks prior to surgery.

11. In a further preferred embodiment, the 4-OHA or ester thereof is present in the pharmaceutical composition referred to in any one of items 6 to 10 in an amount of from about 1.5 wt.-% to about 4 wt.-%, preferably in an amount of from about 2.0 wt.-% to about 3.0 wt.-%, more preferably in an amount of about 2.5 wt.-%, based on the amount of the pharmaceutical composition.

12. In a further preferred embodiment, the pharmaceutical composition referred to in any one of items 6 to 11 is applied topically once or twice a day.

13. The present invention also refers to 4-OHA or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N wherein the ester is formed with the 4-OH (hydroxyl) group and is used as prodrug which upon administration is metabolized, leaving the uncoupled 4-OH group, for use in a pre-surgical topical treatment of ERβ-positive and optionally additionally androgen receptor (AR) positive breast cancer tissue.

### Detailed description of the invention

The present invention is now described in more detail by preferred embodiments and examples, which are however presented for illustrative purpose only and shall not be understood as limiting the scope of the present invention in any way.

In the context of ERβ-positive breast cancer, the present inventors have now unexpectedly found that by topically administering a steroid compound as a prodrug, which upon metabolization exhibits a high selectivity for ERβ and at the same time no or only very less selectivity for ERα, and additionally binds to the androgen receptor (AR) which is optionally also expressed by the breast cancer tissue, a significant reduction of the tumor size can be achieved.

The pharmacologically active substances result from metabolization of the prodrug 4-hydroxyandrost-4-ene-3,17-dione (4-OHA) of formula I or ester thereof, if said prodrug is applied topically to the affected ERβ-positive, AR-receptor-positive, cancerous tissue (breast cancer tissue).

It has been found by the present inventors that upon topical application of 4-OHA (or ester thereof), metabolization of 4-OHA takes place in the fatty tissue of the breast and in the tumor tissue. The metabolization is e.g. mediated by aldo-keto-reductases, which reduce keto-groups that are present in the prodrug. As found by the present inventors, an enzyme that reduces 4-OHA to 4-OHT is AKR1C3 (Aldo-keto reductase family 1 member C3). Due to the presence of (only) one OH-group (compared to the at least two OH-groups of the metabolites) the transdermal penetration of 4-OHA or its ester is improved compared to the transdermal penetration of the metabolites themselves. An example of a metabolite is 4-OHEPI (4-beta-hydroxy-epiandrosterone), 4-OHBA, or 4-OHT.

By topically applying said prodrug (steroid compound) or its ester to ERβ positive and optionally AR positive breast cancer tissue, a reduction of the tumor size can be achieved. As a result of this size reduction, a surgical removal of the breast tumor tissue can be carried out, while at the same time maintaining the affected breast to a greater extent than without the treatment. In other words, by applying the prodrug, a complete removal of the affected breast can often be avoided. Instead, a breast-conserving surgery can be performed. Thus, in the context of the present invention, the prodrug is applied prior to a planned surgical removal of the breast tumor tissue.

Additionally, due to the lower number of OH groups of the prodrug compared to the number of OH groups of the pharmacologically active substance, topical application of the prodrug 4-OHA (or ester thereof) to the affected region (breast) is improved compared to the topical application of the respective pharmacologically active substance, as the skin penetration of the prodrug (or ester thereof) is enhanced.

In particular, it has been found by the present inventors that the hydroxyl-group in C4-position and the hydroxyl-group in C17-position of the metabolite(s) of the prodrug of Formula I or esters thereof respectively play an essential role in fulfilling the object of the present invention:
The hydroxyl-group in C4-position of the metabolized steroid compound enables the resulting pharmacologically active substance to differentiate more precisely between ERα and ERβ expressed by the breast tumor tissue, compared e.g. to beta-adiol that does not exhibit a hydroxyl-group at position C4. As was found out, the metabolite of the present invention exhibiting a hydroxyl-group in C4-position exhibits a binding affinity towards ERβ that is significantly higher compared to its binding affinity towards ERα. For example, the affinity of the metabolite 4-OHBA towards ERβ is about 10 times higher than its affinity towards ERα. Additionally it has been found that by binding to ERβ, the expression of ERβ in breast cancer tumor tissue is enhanced. This enhanced ERβ-expression in turn results in an increase in tumor suppression.

Besides the above disclosed beneficial effect of the OH group at C4-position with regard to the improved affinity towards ERβ (and not towards ERα), there are further beneficial properties based on this specific position of the OH group in the steroid compound of Formula I: The metabolism is strongly accelerated, because the steroid compound 4-OHA is no substrate for the enzyme 5α-reductase which catalyses the conversion of testosterone to dihydrotestosterone (DHT), the affinity towards the androgen receptor (AR) is enhanced; and binding towards SHBG (sex hormone-binding globulin) is inhibited.

It has been found that not only the binding to ERβ, but also the binding to the androgen receptor (AR) results in an increased expression of ERβ. Additionally, binding to the AR can result in a decrease of cell proliferation, or even in an inhibition thereof. This is specifically beneficial if the AR is expressed by a tumor cell, e.g. a breast cancer tumor cell.

The binding of the steroid compound of the present invention to the AR is mediated by the OH-group at position C17. In other words, the hydroxyl-group at C17-position has the effect that the steroid compound additionally has the capability of binding the androgen receptor (AR). This, in turn, results in an activation of AR-mediated signalling pathways, thereby finally evoking an end (or at least reduction) of cellular proliferation.

Thus, the present invention refers to the steroid compound 4-hydroxyandrost-4-ene-3,17-dione (4-OHA) of formula I 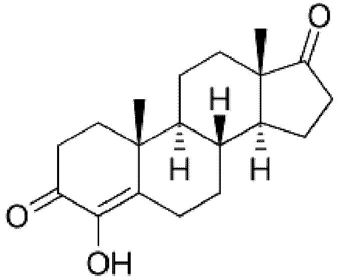 Formula I, or esters thereof, with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group,
for use in a method of treating a patient suffering from estrogen-receptor β (ERβ) positive and optionally androgen receptor (AR) positive breast tumor, wherein said steroid compound is applied topically to the patient. Preferably, the treatment takes place prior to a planned surgical removal of breast tumor tissue (pre-surgical treatment).

It is assumed that the prodrug is metabolized to the pharmaceutically active compound(s) which has/have a hydroxyl-group in at least C4-position and C17-position, for example 4-hydroxytestosterone: 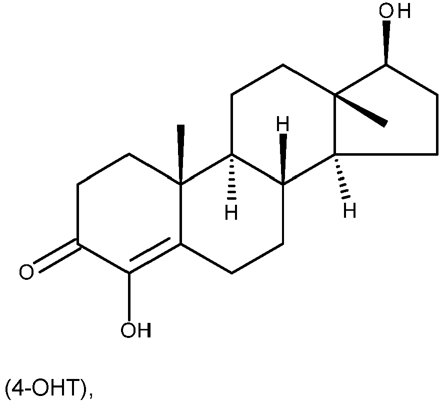 and

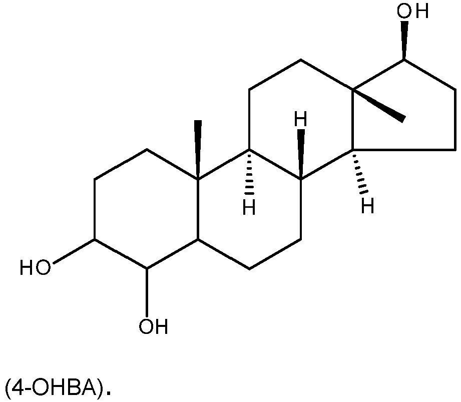

By treating a patient suffering from ERβ positive and preferably also AR positive breast cancer with said prodrug, the tumor tissue is significantly reduced upon completion of the treatment. As a consequence, the tumor tissue is reduced to such an extent compared to the size of the tumor tissue at the beginning of the treatment that a subsequent operation can be carried out in a breast conserving manner. In other words, a complete removal of the affected breast can be avoided.

Thus, in one embodiment, the pre-surgical treatment comprises pre-surgical reduction of the breast tumor tissue.

If not known from other information or data, receptor status of target cells or target tissues with respect to AR and/or ERβ (and also ERα) can be determined and, if desired, quantified by standard methods known to a person skilled in the art. Such standard methods include immunoassays involving AR- and/or ER-specific antibodies, DNA and/or RNA hybridization assays or PCR amplification tests involving AR- and/or ER- specific nucleic acid probes.

In a preferred embodiment, the tumor tissue of the affected breast is reduced by at least 20 vol.-%, preferably by at least 30 vol.-%, more preferably by at least 40 vol.-%, even more preferably by at least 50 vol.-%, compared to the size of the breast tumor tissue at the beginning of the treatment. The size of the tumor tissue can be determined by any suitable mean that is known to a skilled person, such as by ultrasound, X-rays, or mammography. In one embodiment, the above indicated reduction of the tumor volume can be arrived at after a treatment of 40 days.

In a further aspect, the metabolized steroid compound 4-OHA according to the present invention, when comparing its binding affinity towards estrogen-receptor beta (ERβ) with its binding affinity towards estrogen-receptor alpha (ERα), respectively expressed by the breast tumor tissue, exhibits an increased binding affinity towards estrogen-receptor beta (ERβ) expressed by the breast tumor tissue. In a preferred embodiment, the pharmacologically active **substance** exhibits a binding affinity towards ERβ that is at least 3 times, preferably at least 4, 5, or 6 times, more preferably at least 9 times and most preferably at least 10 times higher compared to the biding affinity towards ERβ.

Hence, the metabolized steroid compound of Formula I of the present invention exhibits a binding affinity towards ERβ, mediated by C4-OH group, and preferably towards AR, mediated by C17-OH group.

In an embodiment, the binding affinity specific to AR is in a range of IC₅₀ ≤ 500nM, such as IC₅₀ ≤ 100nM or IC₅₀ ≤ 50nM, wherein IC₅₀ is defined as the concentration of the compound required to reduce specific binding of a reference compound, 5α-dihydrotestosterone (DHT), by 50%. The IC₅₀ - values can be determined by known methods using radioactively labelled DHT as reference compound, for example by a standard dextran-coated charcoal adsorption method as described by Raynaud et al., J. Steroid Biochem. 6, 615-622 (1975), using 1 nM reference concentrations of radiolabeled [³H]-DHT, or by similar IC₅₀ determination methods described in the literature. Because the concentration of AR in the target cell is very low, typically in the nanomolar range approximately, differences in binding constants to the order contemplated in the present invention are significant.

The steroid compound of the present invention optionally additionally exhibits, upon metabolization into a pharmaceutically active substance, androgen-receptor (AR) binding affinity. In breast cancer cells which are AR-positive, this binding results in an increase of ERβ expression, and at the same time cell proliferation is inhibited or at least reduced, preferably significantly reduced.

Moreover, preferably the compounds of the present invention are not substrates for 5α-reductase. As these substances cannot be that easily metabolised as, for example, testosterone, their inhibitory effect on the tumour cell is greatly facilitated and they additionally exert an apoptotic effect on the tumour cell itself.

Since the compounds of the present invention are AR specific compounds, but are not metabolized by aromatase, they can fully exert their actions and effects associated with the specifically defined therapeutic uses via AR.

In a preferred embodiment, the pharmacologically active substance is selected from the group consisting of 4-Hydroxy-5-alpha-androstane-3-beta,17beta-diol (4-OHBA) and 4-Hydroxytestosterone (4-OHT); preferably, the pharmacologically active substance is 4-OHBA.

In experiments carried out with the compounds of the present invention it could be shown that the steroid compound according to Formula I (4-OHA) or ester thereof had excellent skin penetration capabilities so that a sufficient tumour inhibition, or size reduction, respectively, could be achieved by simple topic administration of e.g. an ointment, lotion or cream etc. comprising a therapeutically or prophylactically , preferably therapeutically, effective amount of this steroid compound according to the present invention to an area of a patient in need of treatment. After topical administration, the compound(s) penetrate through the skin and concentrate in the fatty tissue.

Thus, in a further aspect, the present invention refers to a pharmaceutical composition suitable for topical application comprising the steroid compound 4-Hydroxyandrost-4-ene-3,17-dione (4-OHA) of formula I 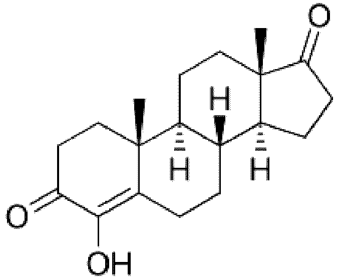 Formula I or esters thereof, with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group,
and pharmaceutically acceptable carrier(s) and/or excipient(s), for use in the treatment of a patient suffering from estrogen-receptor β (ERβ) positive and optionally androgen receptor (AR) positive breast tumor, wherein the treatment takes place prior to a planned surgical removal of the breast tumor tissue.

In a preferred embodiment, the pre-surgical treatment comprises the topical application of the pharmaceutical composition in an amount effective for reducing the breast tumor tissue. This amount can be adapted individually to a patient, dependent on the size of the tumor at the beginning of the therapy.

In a further embodiment, the prodrug 4-OHA or its ester is present in the pharmaceutical composition in an amount of from about 1.5 wt.-% to about 4 wt.-%, preferably in an amount of from about 2.0 wt.-% to about 3.0 wt.-%, more preferably in an amount of about 2.5 wt.-%, based on the amount of the pharmaceutical composition.

As disclosed elsewhere herein, administering topically the prodrug 4-OHA to a patient in need thereof can result in a reduction of the tumor size, to the effect that the breast cancer tissue can be removed breast conserving, that is, a complete removal of the affected breast can be avoided. Hence, this pre-surgical treatment is carried out over a period of time that is sufficient for effecting the reduction of the breast tumor tissue to a size that allows for the breast-preserving surgical removal of the breast tumor tissue.

In a preferred embodiment, the pre-surgical treatment is carried out for a period of time of up to 6 months, or up to 5, 4, 3, or 2 months.

It is also possible that a reduction of the breast tumor tissue to a size that allows for the breast-preserving surgical removal can be effected after a pre-surgical treatment of 11 weeks, or 10, 9, 8, 7, 6, 5, 4, 3, or 2 weeks.

In a further embodiment, the pre-surgical treatment starts no later than 6 months, or no later than 5, 4, 3, or 2 months, prior to the planned surgery, that is, the breast-conserving surgical removal of the breast tumor tissue.

It is also possible that the pre-surgical treatment starts no later than 11 weeks, or 10, 9, 8, 7, 6, 5, 4, 3, or 2 weeks, prior to the planned surgery.

It is also possible that the pre-surgical treatment starts no later than 8 weeks prior to a planned surgery, preferably no later than 6 weeks or 5 weeks prior to surgery.

By applying the prodrug (or the pharmaceutical composition comprising the prodrug, respectively) locally and topically to the skin, the prodrug is transdermally absorbed and thus brought locally to the intended site of action. The pharmacologically active substance concentrates in the periductal fatty tissue. In a long-term treatment, the fatty matter of the treated breast is markedly reduced. This reduction decreases the quantity of oestrogen-forming cells having oestrogen-forming competence. The lipophilicity and hydrophobicity of the pharmacologically active substance has the result that the pharmacologically active substance is exclusively concentrated locally in the fatty tissue and thus cannot display any significant systemic action.

In the present invention, the pharmaceutical composition, or medicament, respectively, is applied topically on or in the vicinity of the intended site of action. According to the invention, the result achieved is an adequate local pharmacologically active substance level in the tissue in the diseased tissue (in therapy) without a noticeable absorption of the pharmacologically active substance taking place in the blood circulation. The benefit of this embodiment thus not only relies in the topical application per se, but in the local topical application in such a way that the active compound concentrates immediately in the tissue at risk and/or diseased tissue, and not indirectly via the blood circulation.

For topical administration, the pharmaceutical composition or medicament is preferably formulated as an ointment, cream, gel, emulsion or lotion. Formulation as a powder or oil is also conceivable. Formulation bases are familiar to the person skilled in the art from the cosmetic and pharmaceutical industry and do not need to be explained here in greater detail. For example, vegetable oils and fats such as almond oil, peanut oil, olive oil, peach kernel oil, castor oil, plant extracts, ethereal oils; furthermore vegetable waxes and synthetic and animal oils, fats or waxes; lecithin, lanolin alcohols, carotene, fragrances, mono- or polyhydric alcohols, urea, preservatives and colourants etc. can be used. Formulation as an oil-in-water or water-in-oil emulsion is preferred.

The content of the prodrug of the present invention of a suitable medicament can be between 0.0001 and 20% by weight, preferably 0.6% until 10% by weight, further preferably 1 and 5% by weight, of the compound according to the invention. A customary range is 0.6 to 5% by weight.

If substances are admixed to promote skin penetration, their content, when using hyaluronidases, can be, for example, between 0.01 and 1% by weight, preferably 0.05 and 0.2% by weight, when using dimethylsorbitol or DMSO between 1 and 25% by weight, preferably 5 and 10% by weight.

Owing to the primary target disease to be treated as described above, a local topic treatment is predominantly carried out with female patients and particularly on breast skin of female patients, although the same therapeutic principle applies also to male breast cancer.

Finally, the present invention also refers to 4-OHA or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group, for use in a pre-surgical topical treatment of ERβ-positive breast cancer tissue.

### Figures

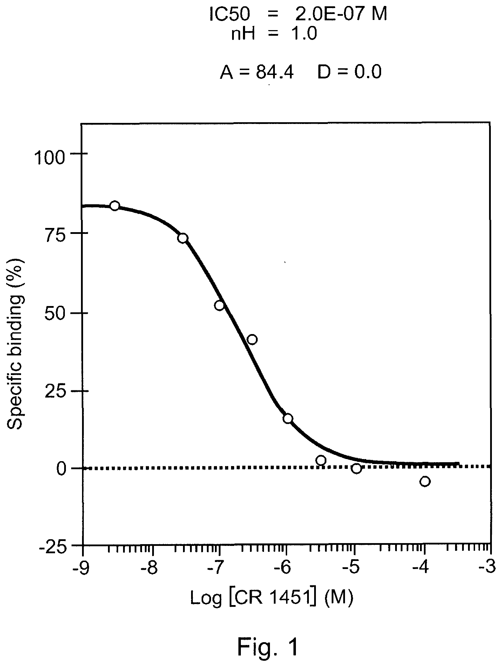

Figure 1 is a diagram showing the specific binding in [%] according to the invention.

The following examples illustrate, but do not limit the invention.

### Examples

### 1. Observation of shrinking of breast tumor tissue

1.1 Patients that were diagnosed (by mammography) with ERβ positive breast cancer tumors with a maximum size of 2 cm were treated with the pharmaceutical composition comprising the prodrug of the present invention: The prodrug was applied topically to the affected breast.

Already after three weeks of treatment, a reduction in tumor size could be observed.

1.2 Application of 4 ml of a crème (2 times/day) comprising 2.5wt.-% 4-OHA to a breast suffering from an ER positive breast cancer. Already after 3-5 weeks, the size of the tumor was significantly decreased.

### 2. Comparison of treatment success of Anastrozol and Letrozol with the prodrug of the present invention

Studies were carried out on patients suffering from ERβ positive breast cancer.

First, the patients were treated with non-steroidal aromatase-inhibitors Anastrozol or Letrozol, the treatment was carried out according to the respective current treatment guidelines. However, the respective treatment was not successful.

Then, said patients were treated with the pharmaceutical composition comprising the prodrug according to the present invention, that is, the prodrug (4-OHA) was applied topically to the affected breast.

It was observed that in 50% of the patients treated in this way, a stabilisation of the disease over 6 months could be reached.

## Claims

1. Steroid compound 4-hydroxyandrost-4-ene-3,17-dione (4-OHA) of formula I 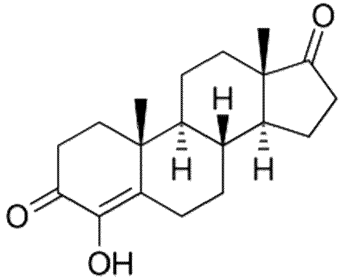
Formula I, or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N,
wherein the ester is formed with the 4-OH (hydroxyl) group and is used as prodrug which upon administration is metabolized, leaving the uncoupled 4-OH group,
for use in a method of treating a patient suffering from estrogen-receptor β (ERβ) positive and optionally androgen receptor (AR) positive breast tumor, wherein said steroid compound is applied topically to the patient.

2. The steroid compound 4-OHA or said esters thereof for use according to claim 1, wherein the treatment takes place prior to a planned surgical removal of breast tumor tissue, and/or wherein the treatment is for reducing the breast tumor tissue.

3. The steroid compound 4-OHA or said esters thereof for use according to claim 1 or 2, wherein upon topical application the prodrug is metabolized to a pharmaceutically active substance, and wherein said 4-OHA is metabolized to a pharmacologically active substance which has at least a hydroxyl-group in C4-position and C17-position.

4. The steroid compound 4-OHA or said esters thereof for use according to any of claims 1 to 3, wherein the 4-OHA is metabolized to a pharmacologically active substance which has a higher binding affinity towards estrogen-receptor beta (ERβ) as compared with its binding affinity towards estrogen-receptor alpha (ERα), which are both expressed by the breast tumor tissue, wherein said treatment results in agonistic binding of ERβ, preferably wherein said pharmacologically active substance exhibits a binding affinity towards ERβ that is at least 3 times, preferably at least 4, 5, or 6 times, more preferably at least 9 times and most preferably at least 10 times higher compared to the binding affinity towards ERα.

5. A pharmaceutical composition suitable for topical application comprising the steroid compound 4-hydroxyandrost-4-ene-3,17-dione (4-OHA) of formula I 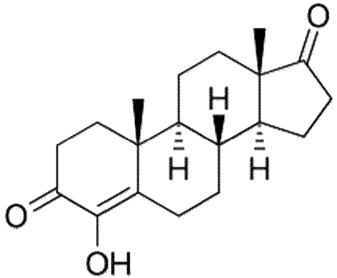
Formula I, or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N,
wherein the ester is formed with the 4-OH (hydroxyl) group and is used as prodrug which upon administration is metabolized, leaving the uncoupled 4-OH group,
and pharmaceutically acceptable carrier(s) and/or excipient(s),
for use in a method of treating a patient suffering from estrogen-receptor β (ERβ) positive and optionally androgen receptor (AR) positive breast tumor, wherein the treatment takes place prior to a planned surgical removal of the breast tumor tissue.

6. The pharmaceutical composition for use according to the preceding claim, wherein the pre-surgical treatment comprises the topical application of the pharmaceutical composition in an amount effective for reducing the breast tumor tissue.

7. The pharmaceutical composition for use according to claim 5 or 6, wherein the pre-surgical treatment is carried out over a period of time that is sufficient for effecting the reduction of the breast tumor tissue to a size that allows for the breast-preserving surgical removal of the breast tumor tissue.

8. The pharmaceutical composition for use according to any of claims 5 to 7, wherein the tumor tissue is significantly reduced upon completion of the treatment, preferably the tumor tissue is reduced by at least 20 vol.-%, preferably by at least 30 vol.-%, more preferably by at least 40 vol.-%, even more preferably by at least 50 vol.-%, compared to the size of the breast tumor tissue at the beginning of the treatment, as determined by mammography, X-ray or ultrasound, preferably as determined by mammography.

9. The pharmaceutical composition for use according to any of claims 5 to 8, wherein the pre-surgical treatment starts no later than 6 months, or no later than 5, 4, 3, or 2 months, prior to the planned surgery, that is, the breast-preserving surgical removal of the breast tumor tissue.

10. The pharmaceutical composition for use according to any of claims 5 to 9, wherein the 4-OHA or said ester thereof is present in the pharmaceutical composition in an amount of from about 0.01 wt.-% to about 4 wt.-%, preferably in an amount of from about 2.0 wt.-% to about 3.0 wt.-%, more preferably in an amount of about 2.5 wt.-%, based on the weight of the pharmaceutical composition.

11. The pharmaceutical composition for use according to any of claims 5 to 10, wherein the pharmaceutical composition is applied topically once or twice a day.

12. 4-OHA or esters thereof with straight chain, branched chain, cyclic and/or aromatic, substituted or unsubstituted hydrocarbon groups with 1-12 carbon atoms, optionally the carbon atoms of the hydrocarbon groups are substituted by 1-3 heteroatoms selected from O, S, and N, wherein the ester is formed with the 4-OH (hydroxyl) group and is used as prodrug which upon administration is metabolized, leaving the uncoupled 4-OH group, for use in a pre-surgical topical treatment of ERβ-positive breast cancer tissue.

## Patentansprüche

1. Steroidverbindung 4-Hydroxyandrost-4-en-3,17-dion (4-OHA) der Formel I 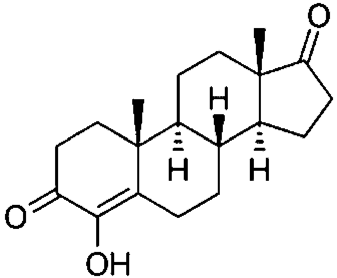
Formel I oder Ester davon mit geradkettigen, verzweigtkettigen, cyclischen und/oder aromatischen, substituierten oder unsubstituierten Kohlenwasserstoffgruppen mit 1-12 Kohlenstoffatomen, wobei optional die Kohlenstoffatome der Kohlenwasserstoffgruppen mit 1-3 Heteroatomen, ausgewählt aus O, S und N, substituiert sind,
wobei der Ester mit der 4-OH(Hydroxyl)-Gruppe gebildet wird und als Prodrug verwendet wird, das bei Verabreichung metabolisiert wird, wobei die ungekoppelte 4-OH-Gruppe zurückbleibt, zur Verwendung in einem Verfahren zur Behandlung eines Patienten, der an Östrogenrezeptor-β (ERβ)-positivem und optional Androgenrezeptor-(AR)-positivem Brusttumor leidet, wobei die Steroidverbindung topisch auf den Patienten aufgebracht wird.

2. Steroidverbindung 4-OHA oder die Ester davon zur Verwendung nach Anspruch 1, wobei die Behandlung vor einer geplanten chirurgischen Entfernung von Brusttumorgewebe stattfindet und/oder wobei die Behandlung zur Reduktion des Brusttumorgewebes dient.

3. Steroidverbindung 4-OHA oder die Ester davon zur Verwendung nach Anspruch 1 oder 2, wobei das Prodrug bei topischer Anwendung zu einer pharmazeutisch aktiven Substanz metabolisiert wird und wobei die 4-OHA zu einer pharmakologisch aktiven Substanz metabolisiert wird, die mindestens eine Hydroxylgruppe in C4-Position und C17-Position aufweist.

4. Steroidverbindung 4-OHA oder die Ester davon zur Verwendung nach einem der Ansprüche 1 bis 3, wobei die 4-OHA zu einer pharmakologisch aktiven Substanz metabolisiert wird, die eine höhere Bindungsaffinität zu Östrogenrezeptor-beta (ERβ) im Vergleich zu ihrer Bindungsaffinität zu Östrogenrezeptor-alpha (ERα) aufweist, die beide durch das Brusttumorgewebe exprimiert werden, wobei die Behandlung zu einer agonistischen Bindung von ERβ führt, wobei die pharmakologisch aktive Substanz vorzugsweise eine Bindungsaffinität zu ERβ aufweist, die mindestens 3-mal, vorzugsweise mindestens 4-, 5- oder 6-mal, mehr bevorzugt mindestens 9-mal und am meisten bevorzugt mindestens 10-mal höher im Vergleich zu der Bindungsaffinität zu ERα ist.

5. Pharmazeutische Zusammensetzung, die zur topischen Anwendung geeignet ist, umfassend die Steroidverbindung 4-Hydroxyandrost-4-en-3,17-dion (4-OHA) der Formel I 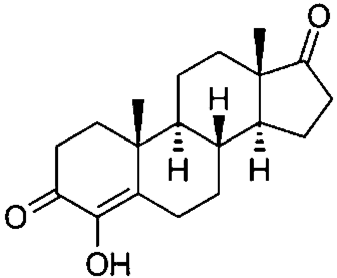
Formel I oder Ester davon mit geradkettigen, verzweigtkettigen, cyclischen und/oder aromatischen, substituierten oder unsubstituierten Kohlenwasserstoffgruppen mit 1-12 Kohlenstoffatomen, wobei optional die Kohlenstoffatome der Kohlenwasserstoffgruppen mit 1-3 Heteroatomen, ausgewählt aus O, S und N, substituiert sind,
wobei der Ester mit der 4-OH(Hydroxyl)-Gruppe gebildet wird und als Prodrug verwendet wird, das bei Verabreichung metabolisiert wird, wobei die ungekoppelte 4-OH-Gruppe zurückbleibt, und einen oder mehrere pharmazeutisch akzeptable Träger und/oder einen oder mehrere pharmazeutisch akzeptable Exzipienten,
zur Verwendung in einem Verfahren zur Behandlung eines Patienten, der an Östrogenrezeptor-β (ERβ)-positivem und optional Androgenrezeptor-(AR)-positivem Brusttumor leidet, wobei die Behandlung vor einer geplanten chirurgischen Entfernung des Brusttumorgewebes stattfindet.

6. Pharmazeutische Zusammensetzung zur Verwendung nach dem vorhergehenden Anspruch, wobei die prächirurgische Behandlung die topische Anwendung der pharmazeutischen Zusammensetzung in einer Menge umfasst, die zur Reduktion des Brusttumorgewebes wirksam ist.

7. Pharmazeutische Zusammensetzung zur Verwendung nach Anspruch 5 oder 6, wobei die prächirurgische Behandlung über einen Zeitraum durchgeführt wird, der ausreichend ist, um die Reduktion des Brusttumorgewebes auf eine Größe zu bewirken, die die brusterhaltende chirurgische Entfernung des Brusttumorgewebes ermöglicht.

8. Pharmazeutische Zusammensetzung zur Verwendung nach einem der Ansprüche 5 bis 7, wobei das Tumorgewebe nach Abschluss der Behandlung signifikant reduziert wird, wobei das Tumorgewebe vorzugsweise um mindestens 20 Vol.-%, vorzugsweise um mindestens 30 Vol.-%, mehr bevorzugt um mindestens 40 Vol.-%, noch mehr bevorzugt um mindestens 50 Vol.-% reduziert wird, im Vergleich zu der Größe des Brusttumorgewebes zu Beginn der Behandlung, wie durch Mammographie, Röntgenstrahlen oder Ultraschall bestimmt, vorzugsweise wie durch Mammographie bestimmt.

9. Pharmazeutische Zusammensetzung zur Verwendung nach einem der Ansprüche 5 bis 8, wobei die prächirurgische Behandlung nicht später als 6 Monate oder nicht später als 5, 4, 3 oder 2 Monate vor der geplanten Operation beginnt, d. h. der brusterhaltenden chirurgischen Entfernung des Brusttumorgewebes.

10. Pharmazeutische Zusammensetzung zur Verwendung nach einem der Ansprüche 5 bis 9, wobei die 4-OHA oder der Ester davon in der pharmazeutischen Zusammensetzung in einer Menge von etwa 0,01 Gew.-% bis etwa 4 Gew.-%, vorzugsweise in einer Menge von etwa 2,0 Gew.-% bis etwa 3,0 Gew.-%, mehr bevorzugt in einer Menge von etwa 2,5 Gew.-%, bezogen auf das Gewicht der pharmazeutischen Zusammensetzung, vorliegt.

11. Pharmazeutische Zusammensetzung zur Verwendung nach einem der Ansprüche 5 bis 10, wobei die pharmazeutische Zusammensetzung ein- oder zweimal täglich topisch aufgebracht wird.

12. 4-OHA oder Ester davon mit geradkettigen, verzweigtkettigen, cyclischen und/oder aromatischen, substituierten oder unsubstituierten Kohlenwasserstoffgruppen mit 1-12 Kohlenstoffatomen, wobei optional die Kohlenstoffatome der Kohlenwasserstoffgruppen mit 1-3 Heteroatomen, ausgewählt aus O, S und N, substituiert sind, wobei der Ester mit der 4-OH (Hydroxyl)-Gruppe gebildet wird und als Prodrug verwendet wird, das bei Verabreichung metabolisiert wird, wobei die ungekoppelte 4-OH-Gruppe zurückbleibt, zur Verwendung in einer prächirurgischen topischen Behandlung von ERβ-positivem Brustkrebsgewebe.

## Revendications

1. Composé stéroïde 4-hydroxyandrost-4-ène-3,17-dione (4-OHA) de formule I 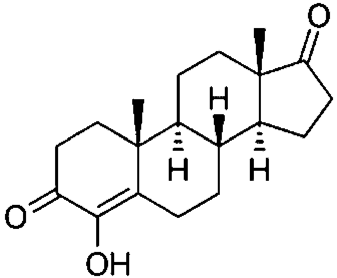
Formule I, ou esters de celui-ci avec des groupes hydrocarbonés à chaîne linéaire, à chaîne ramifiée, cycliques et/ou aromatiques, substitués ou non substitués avec 1 à 12 atomes de carbone, facultativement les atomes de carbone des groupes hydrocarbonés sont substitués par 1 à 3 hétéroatomes choisis parmi O, S et N,
où l'ester est formé avec le groupe 4-OH (hydroxyle) et est utilisé comme promédicament qui, lors de l'administration, est métabolisé, laissant le groupe 4-OH non couplé,
pour une utilisation dans un procédé de traitement d'un patient souffrant d'une tumeur du sein positive au récepteur des œstrogènes β (ERβ) et facultativement positive au récepteur des androgènes (AR), où ledit composé stéroïde est appliqué par voie topique au patient.

2. Composé stéroïde 4-OHA ou lesdits esters de celui-ci pour une utilisation selon la revendication 1, où le traitement a lieu avant un retrait chirurgical planifié du tissu tumoral du sein, et/ou où le traitement est destiné à réduire le tissu tumoral du sein.

3. Composé stéroïde 4-OHA ou lesdits esters de celui-ci pour une utilisation selon la revendication 1 ou 2, où, lors de l'application topique, le promédicament est métabolisé en une substance pharmaceutiquement active, et où ledit 4-OHA est métabolisé en une substance pharmacologiquement active qui a au moins un groupe hydroxyle en position C4 et en position C17.

4. Composé stéroïde 4-OHA ou lesdits esters de celui-ci pour une utilisation selon l'une quelconque des revendications 1 à 3, où le 4-OHA est métabolisé en une substance pharmacologiquement active qui a une affinité de liaison plus élevée envers le récepteur des œstrogènes bêta (ERβ) par rapport à son affinité de liaison envers le récepteur des œstrogènes alpha (ERα), qui sont tous deux exprimés par le tissu tumoral du sein, où ledit traitement conduit à une liaison agoniste de ERβ, de préférence où ladite substance pharmacologiquement active présente une affinité de liaison envers ERβ qui est au moins 3 fois, de préférence au moins 4, 5 ou 6 fois, plus préférablement au moins 9 fois et le plus préférablement au moins 10 fois plus élevée par rapport à l'affinité de liaison envers ERα.

5. Composition pharmaceutique appropriée pour une application topique comprenant le composé stéroïde 4-hydroxyandrost-4-ène-3,17-dione (4-OHA) de formule I 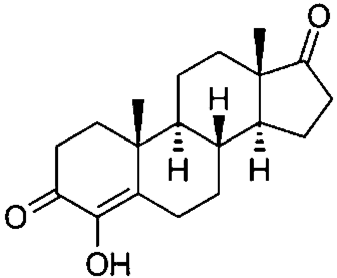
Formule I, ou esters de celui-ci avec des groupes hydrocarbonés à chaîne linéaire, à chaîne ramifiée, cycliques et/ou aromatiques, substitués ou non substitués avec 1 à 12 atomes de carbone, facultativement les atomes de carbone des groupes hydrocarbonés sont substitués par 1 à 3 hétéroatomes choisis parmi O, S et N,
où l'ester est formé avec le groupe 4-OH (hydroxyle) et est utilisé comme promédicament qui, lors de l'administration, est métabolisé, laissant le groupe 4-OH non couplé,
et un(des) support(s) et/ou excipient(s) pharmaceutiquement acceptable(s),
pour une utilisation dans un procédé de traitement d'un patient souffrant d'une tumeur du sein positive au récepteur des œstrogènes β (ERβ) et facultativement positive au récepteur des androgènes (AR), où le traitement a lieu avant un retrait chirurgical planifié du tissu tumoral du sein.

6. Composition pharmaceutique pour une utilisation selon la revendication précédente, où le traitement préchirurgical comprend l'application topique de la composition pharmaceutique en une quantité efficace pour réduire le tissu tumoral du sein.

7. Composition pharmaceutique pour une utilisation selon la revendication 5 ou 6, où le traitement préchirurgical est réalisé sur une période de temps qui est suffisante pour effectuer la réduction du tissu tumoral du sein à une taille qui permet le retrait chirurgical préservant le sein du tissu tumoral du sein.

8. Composition pharmaceutique pour une utilisation selon l'une quelconque des revendications 5 à 7, où le tissu tumoral est considérablement réduit à la fin du traitement, de préférence le tissu tumoral est réduit d'au moins 20 % en volume, de préférence d'au moins 30 % en volume, plus préférablement d'au moins 40 % en volume, encore plus préférablement d'au moins 50 % en volume, par rapport à la taille du tissu tumoral du sein au début du traitement, telle que déterminée par mammographie, rayons X ou ultrasons, de préférence telle que déterminée par mammographie.

9. Composition pharmaceutique pour une utilisation selon l'une quelconque des revendications 5 à 8, où le traitement préchirurgical commence au plus tard 6 mois, ou au plus tard 5, 4, 3, ou 2 mois, avant la chirurgie planifiée, c'est-à-dire le retrait chirurgical préservant le sein du tissu tumoral du sein.

10. Composition pharmaceutique pour une utilisation selon l'une quelconque des revendications 5 à 9, où le 4-OHA ou ledit ester de celui-ci est présent dans la composition pharmaceutique en une quantité d'environ 0,01 % en poids à environ 4 % en poids, de préférence en une quantité d'environ 2,0 % en poids à environ 3,0 % en poids, plus préférablement en une quantité d'environ 2,5 % en poids, sur la base du poids de la composition pharmaceutique.

11. Composition pharmaceutique pour une utilisation selon l'une quelconque des revendications 5 à 10, où la composition pharmaceutique est appliquée par voie topique une ou deux fois par jour.

12. 4-OHA ou esters de celui-ci avec des groupes hydrocarbonés à chaîne linéaire, à chaîne ramifiée, cycliques et/ou aromatiques, substitués ou non substitués avec 1 à 12 atomes de carbone, facultativement les atomes de carbone des groupes hydrocarbonés sont substitués par 1 à 3 hétéroatomes choisis parmi O, S et N, où l'ester est formé avec le groupe 4-OH (hydroxyle) et est utilisé comme promédicament qui, lors de l'administration, est métabolisé, laissant le groupe 4-OH non couplé, pour une utilisation dans un traitement topique préchirurgical d'un tissu de cancer du sein positif à ERβ.
